# EUROPEAN PATENT APPLICATION

(11) **EP 2 340 793 A2**
(43) Date of publication of application: **06.07.2011**
(21) Application number: 11161312.1
(22) Date of filing: 21.06.2005
(51) Int. Cl.: A61F 13/15, D04H 11/08, A61L 15/34, A61L 15/48, A61L 15/42

(54) **Absorbent article with lotion-containing topsheet**

(30) Priority: 21.06.2004 US 581483 P
(62) Divisional of application: 05766062.3
(71) Applicant: The Procter & Gamble Company, Cincinnati, OH 45202 (US)
(72) Inventor: Hammons, John Lee, Hamilton, Cincinnati, OH 45011 (US); Noel, John Richard, Cincinnati, OH 45249 (US); Warren, Raphael, AMBERLEY VILLAGE, OH 45237 (US)
(74) Representative: Kremer, Véronique Marie Joséphine

(57) **Abstract**

A sanitary napkin comprising a topsheet having a body-facing side and comprising a plurality of discrete tufts of fibrous material. The topsheet has a lotion composition applied to at least a portion of the body-facing side thereof. An absorbent core is in fluid communication with the topsheet, the absorbent core having an average thickness of less than about 10 mm, and a free absorbent capacity of from about 4 to about 125 grams per gram.

## Description

### FIELD OF INVENTION

This invention relates to fibrous webs such as nonwoven webs suitable for use as a topsheet in a disposable absorbent article. In particular, this invention relates to fibrous webs treated by mechanical formation to have increased softness or bulk properties, and having a lotion applied thereto.

### BACKGROUND OF THE INVENTION

Disposable absorbent articles such as baby diapers, adult incontinence products, sanitary napkins, pantiliners, hemorrhoid treatment pads, bandages, and the like are well known in the art. Such articles generally have a fluid permeable topsheet, and fluid impermeable backsheet, and an absorbent core sandwiched between the topsheet and the backsheet to absorb and contain body fluid exudates.

In some applications of disposable absorbent articles, such as sanitary napkins and pantiliners, it is desirable to not only absorb body fluids, but to minimize fluid on the body of the wearer. Fluid on the body can be minimized by ensuring that the fluid enters the absorbent article, and does not come back out, such as by being pressed or squeezed out during the normal course of wearing the absorbent article, i.e., by sitting or walkin. While much work has been done in to minimize rewet to the body, there remains a need for a disposable absorbent article that helps keep the users body clean and dry.

Accordingly, there is a disposable absorbent article that helps provide for a clean body benefit in the area of sanitary napkins and pantiliners.

Additionally, there is a need for a method for relatively inexpensively making a disposable absorbent article that helps provide for a clean body benefit in the area of sanitary napkins and pantiliners.

### SUMMARY OF THE INVENTION

A sanitary napkin comprising a topsheet having a body-facing side and comprising a plurality of discrete tufts of fibrous material is disclosed. The topsheet has a lotion composition applied to at least a portion of the body-facing side thereof. An absorbent core is in fluid communication with the topsheet, the absorbent core having an average thickness of less than about 10 mm, and a free absorbent capacity of from about 4 to about 125 grams per gram.

### BRIEF DESCRIPTION OF THE DRAWINGS

FIG. 1 is a perspective view of a web of the present invention.
FIG. 2 is an enlarged view of a portion of the web shown in FIG. 1.
FIG. 3 is a cross-sectional view of section 3-3 of FIG. 2.
FIG. 4 is a plan view of a portion of the web as indicated by 4-4 in FIG. 3.
FIG. 5 is a perspective view of an apparatus for forming the web of the present invention.
FIG. 6 is a cross-sectional depiction of a portion of the apparatus shown in FIG. 5.
FIG. 7 is a perspective view of a portion of the apparatus for forming one embodiment the web of the present invention.
FIG. 8 is an enlarged perspective view of a portion of the apparatus for forming the web of the present invention.

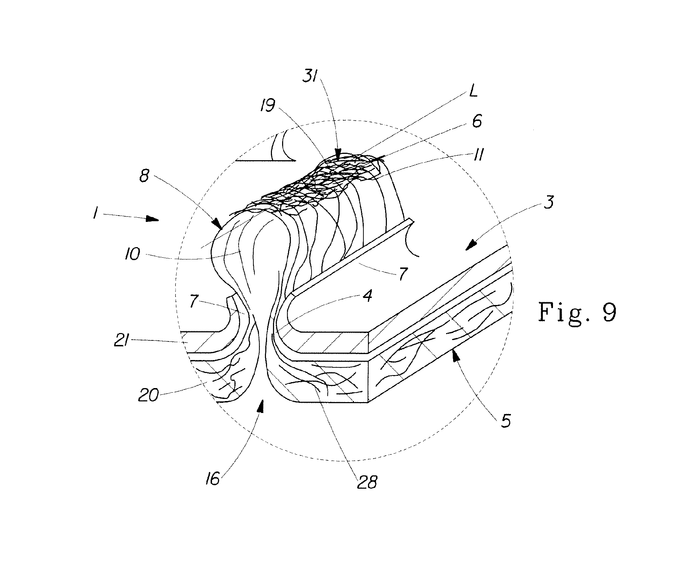

FIG. 9 is an enlarged view of a portion of another embodiment of a web of the present invention.
FIG. 10 is a partial cut away plan view of a sanitary napkin of the present invention.

### DETAILED DESCRIPTION OF THE INVENTION

The present invention can be utilized in any of known disposable absorbent products. In a preferred embodiment, however, the present invention comprises a sanitary napkin intended to be used as a menstrual pad. The sanitary napkin of the present invention comprises at least three components: a topsheet, a lotion applied to the topsheet, and an absorbent core in fluid communication with the topsheet. It has been unexpectedly found that, by using the combination of materials disclosed below, a sanitary napkin of the present invention can provide for a desirable clean body benefit. Specifically, the sanitary napkin of the present invention provides for better fluid acquisition and retention, such that less fluid is left on the body of the wearer, or is squeezed back out of the product onto the body of the wearer.

It has been found that application of a semi-solid lotion material to the top surface of a sanitary napkin can modulate skin properties and conditions of the wearer. It is believed that this is due to the semi-solid lotion melting when the article is worn against the body, and subsequent transferring from the topsheet to the skin of the wearer. In a preferred embodiment, the lotion is a hydrophobic semi-solid lotion, which when applied to the top surface of a sanitary napkin, especially when the sanitary napkin has a hydrophobic surface, can confer a benefit of reducing rewet from the article to the wearer's body, resulting in a drier wearing experience.

In one embodiment, a hydrophobic semi-solid lotion is applied to an absorbent article having a hydrophobic topsheet surface. Because such a lotion can tend to negatively affect the acquisition of the fluid into the absorbent article, resulting in soiling of the wearer's body and/or garments, the lotion is preferably applied in non-continuous patterns, such as in stripes or bands. In another embodiment, a hydrophobic semi-solid lotion is applied to an absorbent article having a hydrophilic topsheet surface.

It has been found that a hydrophobic semi-solid lotion material disposed on the outer, lateral side areas of a sanitary napkin having a hydrophobic topsheet allows for good fluid acquisition, reduced rewet, and reduced residual fluid on the body and transfer to the wearer's body. For example, the lotion can be applied in longitudinal stripes or bands. In one embodiment, the lotion is applied in two 22 mm stripes of longitudinally oriented bands, separated by a gap of about 20 mm free of lotion in the center of the article. Without being bound by theory, it is believed that when used with the topsheet and the core of the present invention, this permits the benefit of reduced rewet while preserving acquisition because the lotion can transfer to the wearers body more efficiently, particularly when the wearer's body is in motion. A hydrophobic coating can help prevent menses from sticking to the body, for example. Further, the lotion can also transfer back to the topmost area of the article. This lotion coating of the previously uncoated surface of the article appears not to compromise the functioning of the article's surface and underlying absorbent materials, as might be the case when the semi-solid is melted and then uniformly applied on the surface by spray or slot coat application.

When the lotion of the present invention is applied as described and worn by the user, it is surprisingly found that only a small amount of the semi-solid lotion material transferred to the non-applied areas of the topsheet lowers rewet, without negatively affecting fluid acquisition. For example, in one embodiment, lotion in the amount of about 7 grams per square meter (gsm) on the areas of the topsheet (e.g., the stripe) where it is applied is adequate for transferring sufficient amounts of lotion to the skin and hair of the wearer. In other embodiments, lotion can be added at a basis weight of from about 8-20 gsm, in one gsm increments. In one embodiment, the lotion can be applied only to the tips of tufts **6** by use of a kiss roll, or printing roll, or the like. Such a topsheet delivers skin care and fluid handling benefits regardless of the underlying absorbent core and other components of the sanitary napkin.

The following description will describe in order: a topsheet of the present invention, a lotion of the present invention, and an absorbent core of the present invention.

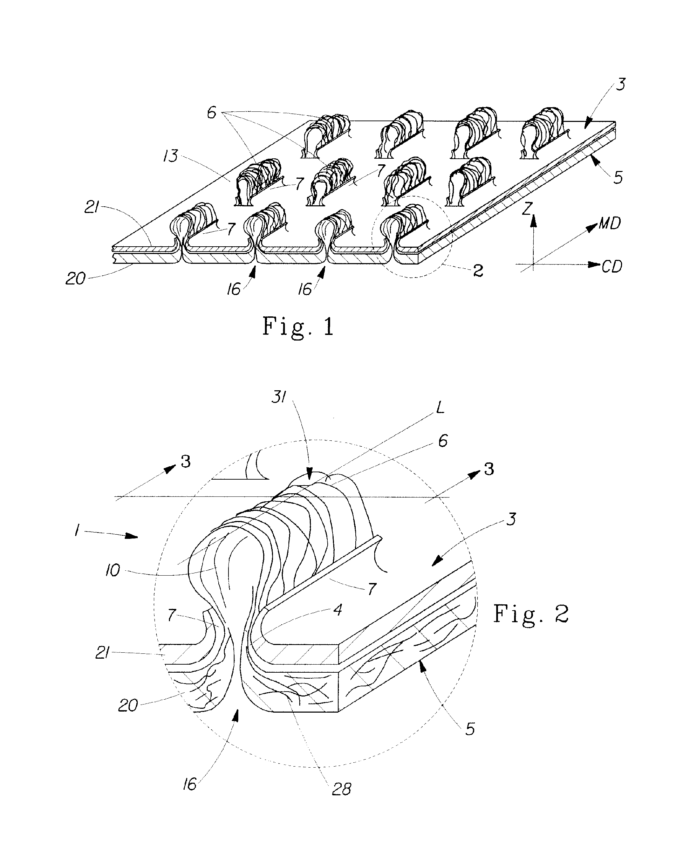

FIG. 1 shows a laminate web **1** suitable for use as a topsheet in the present invention, hereinafter referred to simply as web **1.** Web **1** can comprise one layer, but in a preferred embodiment comprises at least two layers. The layers are referred to herein as generally planar, two-dimensional precursor webs, such as first precursor web **20** and second precursor web **21.** Either precursor web can be a film, a nonwoven, but in a preferred embodiment, both precursor webs are nonwoven webs. Precursor webs **20** and **21** (and any additional webs) can be joined by adhesive, thermal bonding, ultrasonic bonding and the like, but are preferably joined without the use of adhesive or other forms of bonding. As disclosed below, the constituent precursor webs of web **1** can be joined by interlocking mechanical engagement resulting from the formation of tufts **6.**

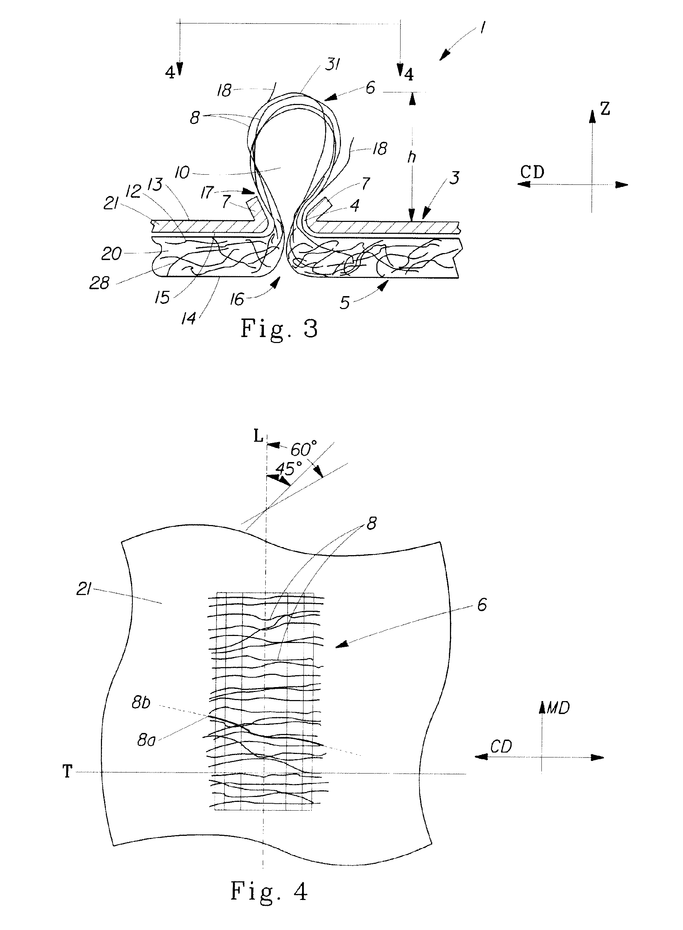

Web **1** has a first side **3** and a second side **5,** the term "sides" being used in the common usage of generally planar two-dimensional webs, such as paper and films that have two sides when in a generally flat condition. Each precursor web **20** and **21** has a first surface **12** and **13,** respectively, and a second surface **14** and **15,** respectively (shown in FIG. 3). Web **1** has a machine direction (MD) and a cross machine direction (CD) as is commonly known in the art of web manufacture. Although the present invention can be practiced with polymer films and woven webs, in a preferred embodiment both precursor webs are nonwoven webs comprised of substantially randomly oriented fibers. By "substantially randomly oriented" is meant that, due to processing conditions of the precursor web, there may be a higher amount of fibers oriented in the MD than the CD, or vice-versa. For example, in spunbonding and meltblowing processes continuous strands of fibers are deposited on a support moving in the MD. Despite attempts to make the orientation of the fibers of the spunbond or meltblown nonwoven web truly "random," usually a slightly higher percentage of fibers are oriented in the MD as opposed to the CD. In a preferred embodiment, first precursor web **20** is a relatively hydrophilic nonwoven web and second precursor web **21** is a nonwoven web is a relatively hydrophobic nonwoven web. For all nonwoven webs, the hydrophobicity or hydrophilicity can be achieved by use of fibers having the proper characteristics, or the precursor webs can be treated to have the desired characteristics.

In one embodiment, first side **3** of web **1** is defined by exposed portions of the first surface **13** of second precursor web **21** and at least one, but preferably a plurality of, discrete tufts **6** which are integral extensions of the fibers of at least first precursor web **20** and preferably both precursor webs. As shown in FIG. 3, each tuft **6** can comprise a plurality of looped, aligned fibers **8** extending through second precursor web **21** and outwardly from the first surface **13** thereof. In another embodiment each tuft **6** can comprise a plurality of non-looped fibers **18** (as shown in FIG. 3) that extend outwardly from the first surface **13.**

As used herein, the term "nonwoven web" refers to a web having a structure of individual fibers or threads which are interlaid, but not in a repeating pattern as in a woven or knitted fabric, which do not typically have randomly oriented fibers. Nonwoven webs or fabrics have been formed from many processes, such as, for example, meltblowing processes, spunbonding processes, hydroentangling, airlaying, and bonded carded web processes, including carded thermal bonding. Fibers can be bicomponent, multicomponent, multiconstituent, and the like, as known in the art. The basis weight of nonwoven fabrics is usually expressed in grams per square meter (gsm). The basis weight of the laminate web is the combined basis weight of the constituent layers and any other added components. Fiber diameters are usually expressed in microns; fiber size can also be expressed in denier, which is a unit of weight per length of fiber. The basis weight of laminate webs suitable for use in the present invention can range from 10 gsm to 500 gsm.

The constituent fibers of nonwoven precursor web **20** or **21** can be comprised of polymers such as polyethylene, polypropylene, polyester, and blends thereof. The fibers can comprise cellulose, rayon, cotton, or other natural materials or blends of polymer and natural materials. The fibers can also comprise a super absorbent material such as polyacrylate or any combination of suitable materials. The fibers can be monocomponent, bicomponent, and/or biconstituent, non-round (e.g., capillary channel fibers), and can have major cross-sectional dimensions (e.g., diameter for round fibers) ranging from 0.1-500 microns. For example, one type of fibers suitable for the nonwoven web includes nanofibers. Nanofibers are described as fibers having a mean diameter of less than 1 micron. Nanofibers can comprise all of the fibers in a nonwoven web or a portion of the fibers in a nonwoven web. The constituent fibers of the nonwoven precursor web may also be a mixture of different fiber types, differing in such features as chemistry (e.g. PE and PP), components (mono- and bi-), denier (micro denier and >20 denier), shape (i.e. capillary and round) and the like. The constituent fibers can range from about 0.1 denier to about 100 denier.

As used herein, "spunbond fibers" refers to small diameter fibers which are formed by extruding molten thermoplastic material as filaments from a plurality of fine, usually circular capillaries of a spinneret with the diameter of the extruded filaments then being rapidly reduced. Spunbond fibers are generally not tacky when they are deposited on a collecting surface. Spunbond fibers are generally continuous and have average diameters (from a sample of at least 10) larger than 7 microns, and more particularly, between about 10 and 40 microns.

As used herein, the term "meltblowing" refers to a process in which fibers are formed by extruding a molten thermoplastic material through a plurality of fine, usually circular, die capillaries as molten threads or filaments into converging high velocity, usually heated, gas (for example air) streams which attenuate the filaments of molten thermoplastic material to reduce their diameter, which may be to microfiber diameter. Thereafter, the meltblown fibers are carried by the high velocity gas stream and are deposited on a collecting surface, often while still tacky, to form a web of randomly dispersed meltblown fibers. Meltblown fibers are microfibers which may be continuous or discontinuous and are generally smaller than 10 microns in average diameter.

As used herein, the term "polymer" generally includes, but is not limited to, homopolymers, copolymers, such as for example, block, graft, random and alternating copolymers, terpolymers, etc., and blends and modifications thereof. In addition, unless otherwise specifically limited, the term "polymer" includes all possible geometric configurations of the material. The configurations include, but are not limited to, isotactic, atactic, syndiotactic, and random symmetries.

As used herein, the term "monocomponent" fiber refers to a fiber formed from one or more extruders using only one polymer. This is not meant to exclude fibers formed from one polymer to which small amounts of additives have been added for coloration, antistatic properties, lubrication, hydrophilicity, etc. These additives, for example titanium dioxide for coloration, are generally present in an amount less than about 5 weight percent and more typically about 2 weight percent.

As used herein, the term "bicomponent fibers" refers to fibers which have been formed from at least two different polymers extruded from separate extruders but spun together to form one fiber. Bicomponent fibers are also sometimes referred to as conjugate fibers or multicomponent fibers. The polymers are arranged in substantially constantly positioned distinct zones across the cross-section of the bicomponent fibers and extend continuously along the length of the bicomponent fibers. The configuration of such a bicomponent fiber may be, for example, a sheath/core arrangement wherein one polymer is surrounded by another, or may be a side-by-side arrangement, a pie arrangement, or an "islands-in-the-sea" arrangement.

As used herein, the term "biconstituent fibers" refers to fibers which have been formed from at least two polymers extruded from the same extruder as a blend. Biconstituent fibers do not have the various polymer components arranged in relatively constantly positioned distinct zones across the cross-sectional area of the fiber and the various polymers are usually not continuous along the entire length of the fiber, instead usually forming fibrils which start and end at random. Biconstituent fibers are sometimes also referred to as multiconstituent fibers.

As used herein, the term "non-round fibers" describes fibers having a non-round cross-section, and includes "shaped fibers" and "capillary channel fibers." Such fibers can be solid or hollow, and they can be tri-lobal, delta-shaped, and are preferably fibers having capillary channels on their outer surfaces. The capillary channels can be of various cross-sectional shapes such as "U-shaped", "H-shaped", "C-shaped" and "V-shaped". One preferred capillary channel fiber is T-401, designated as 4DG fiber available from Fiber Innovation Technologies, Johnson City, TN. T-401 fiber is a polyethylene terephthalate (PET polyester).

As used herein, the term "integral" as in "integral extension" when used for the tufts **6** refers to fibers of the tufts **6** having originated from the fibers of the precursor webs. For example, fibers in tufts **6** can be integral with, i.e., originated in, first precursor web **20.** Therefore, the looped fibers **8** and non-looped fibers **18** of tufts **6,** can be plastically deformed and extended fibers of the first precursor web **20,** and are, therefore, integral with first precursor web **20.** As used herein, "integral" is to be distinguished from fibers introduced to or added to a separate precursor web for the purpose of making tufts, as is commonly done in conventional carpet making, for example.

The number, spacing, and dimensions of tufts **6** can be varied to give varying texture to first side **3** of web **1.** For example, if tufts **6** are sufficiently closely spaced the first side **3** of web **1** can have a terry cloth-like feel. Alternatively, tufts **6** can be arranged in patterns such as lines or filled shapes to create portions of a laminate web having greater texture, softness, bulk, absorbency or visual design appeal. For example, when tufts **6** are arranged in a pattern of a line or lines, the tufts can have the appearance of stitching. Tufts **6** can also be arranged to form specific shapes, such as designs, words or logos. Such shapes can be used, for example, on laminates useful for hotel bath towels or robes which can have the name or logo of the hotel formed thereon. Likewise, the size dimensions, such as the height, length and width of individual tufts **6** can be varied. Single tufts can be as long as about 3 cm in length and can be made alone or dispersed among tufts of various sizes.

First precursor web **20** can be a fibrous woven or nonwoven web comprising fibers having sufficient elongation properties to have portions formed into tufts **6** as described more fully below. Tufts are formed by urging fibers out-of-plane in the Z-direction at discrete, localized, portions of first precursor web **20.** The urging out-of-plane can be due to fiber displacement, i.e., the fiber is able to move relative to other fibers and be "pulled," so to speak, out-of-plane. More often, however, for most nonwoven first precursor webs **20,** the urging out-of-plane is due to the fibers of tufts **6** having been at least partially plastically stretched and permanently deformed to form tufts **6.** Therefore, in one embodiment, depending on the desired height of tufts **6,** the constituent fibers of a nonwoven first precursor webs **20** can exhibit an elongation to break of at least about 5%, more preferably at least about 10%, more preferably at least about 25%, more preferably at least about 50%, and more preferably at least about 100%. Elongation to break can be determined by simple tensile testing, such as by use of Instron tensile testing equipment, and can generally be found on material data sheets from suppliers of such fibers or webs.

It can be appreciated that a suitable precursor webs should comprise fibers capable of experiencing sufficient plastic deformation and tensile elongation, or are capable of sufficient fiber mobility, such that looped fibers **8** are formed. However, it is recognized that a certain percentage of fibers urged out of the plane of the first surface **12** will not form a loop, but instead will break and form loose ends. Such fibers are referred to herein as "loose" fibers or "loose fiber ends" **18** as shown in FIG. 3. Loose fiber ends **18** are not necessarily undesirable for the present invention, and in some embodiments, most or all of the fibers of tufts **6** can be loose fiber ends **18.** Loose fiber ends **18** can also be the result of forming tufts **6** from nonwoven webs consisting of, or containing, cut staple fibers. In such a case, some number of the staple fiber ends may protrude into the tuft **6,** depending upon such things as the number of staple fibers in the web, the staple fiber cut length, and the height of the tufts. In some instances, it may be desired to use a blend of fibers of different lengths in a precursor web or fibers of different lengths in different layers. This may be able to selectively separate the longer fibers from the shorter fibers. The longer fibers may predominately form the tuft **6** while the shorter fibers predominately remain in the portion of the web not forming the tuft 6. An exemplary mixture of fiber lengths can include fibers of approximately 2 to 8 centimeters for the longer fibers and less than about 1 centimeter for the shorter fibers.

First precursor web **20** can be a fibrous woven or nonwoven web comprising elastic or elastomeric fibers. Elastic or elastomeric fibers can be stretched at least about 50% and return to within 10% of their original dimension. Tufts **6** can be formed from elastic fibers if the fibers are simply displaced due to the mobility of the fiber within the nonwoven, or if the fibers are stretched beyond their elastic limit and are plastically deformed.

Second precursor web **21** can be virtually any web material, the only requirement being that it have sufficient integrity to be formed into the laminate by the process described below. In one embodiment, it can have sufficiently less elongation properties relative to first precursor web **20,** such that upon experiencing the strain of fibers from first precursor web **20** being urged out-of-plane in the direction of second precursor web **21,** second precursor web **21** will rupture, e.g., by tearing due to extensional failure, such that portions of first precursor web **20** can extend through, (i.e., "punch through" so to speak), second precursor web **21** to form tufts **6** on first side **3** of web **1.** In one embodiment second precursor web **21** is a polymer film. Second precursor web **21** can also have sufficient elongation properties to be formed into looped fibers, as described above with respect to first precursor web **20.**

A representative tuft **6** for the embodiment of web **1** shown in FIG. 1 (where the second precursor web **21** is "punched through" by first precursor web) is shown in a further enlarged view in FIG. 2. As shown in FIG. 2 or 3, tuft **6** comprises a plurality of looped fibers **8** that are substantially aligned such that tuft **6** has a distinct linear orientation and a longitudinal axis **L.** Tuft **6** also have a transverse axis **T** generally orthogonal to longitudinal axis **L** in the **MD-CD** plane. In the embodiment shown in FIGS. 1 and 2, longitudinal axis **L** is parallel to the **MD.** In one embodiment, all the spaced apart tufts **6** have generally parallel longitudinal axes **L.** The number of tufts **6** per unit area of web **1,** i.e., the area density of tuft **6**, can be varied from 1 tuft per unit area, e.g., square centimeter to as high as 100 tufts per square centimeter. There can be at least 10, or at least 20 tufts **6** per square centimeter, depending on the end use. In general, the area density need not be uniform across the entire area of web **1,** but tufts **6** can be only in certain regions of web **1,** such as in regions having predetermined shapes, such as lines, stripes, bands, circles, and the like.

As can be appreciated by the description herein, in many embodiments of web **1** openings **4** of second precursor web **21** will have a distinct linear orientation and a longitudinal axis, which is oriented parallel to the longitudinal axis **L** of its corresponding tuft **6.** Likewise, openings **4** will also have a transverse axis generally orthogonal to longitudinal axis in the **MD-CD** plane.

As shown in FIGS. 1-4, tufts **6** can extend through openings **4** in second precursor web **21.** Openings **4** are formed by locally rupturing second precursor web **21** by the process described in detail below. Rupture may involve a simple splitting open of second precursor web **21,** such that opening **4** remains a simple two-dimensional aperture. However, for some materials, such as polymer films, portions of second precursor web **21** can be deflected or urged out-of-plane (i.e., the plane of second precursor web **21)** to form flap-like structures, referred to herein as flap, or flaps, **7.** The form and structure of flaps **7** is highly dependent upon the material properties of second precursor web **21.** Flaps **7** can have the general structure of one or more flaps, as shown in FIGS. 1 and 2. In other embodiments, flap **7** can have a more volcano-like structure, as if the tuft **6** is erupting from the flap **7.** In other embodiments, flaps **7** can virtually completely cover tufts **6,** such that they form a "cap" over tufts **6.**

In one embodiment flaps **7** do not contribute significantly to the material of tufts **6,** and particularly do not contribute significantly to the tactile quality of tufts **6.** In one embodiment, therefore, the laminate web **1** comprises at least two layers (i.e., precursor webs **20** and **21),** but at least one of the layers (i.e., precursor web **21** in FIGS. 1-4) does not significantly affect on the tactile qualities of tufts **6.**

In one embodiment, flaps **7** may extend out of plane significantly, even being as high, so to speak, as the tufts **6** themselves. In this embodiment flaps **7** can cause the tufts **6** to be more resilient and less susceptible to flattening due to compressive or bending forces. In one embodiment, therefore, the laminate web **1** comprises at least two layers (i.e., precursor webs **20** and **21),** and both layers affect the tactile qualities of tufts **6.**

Tufts **6** can comprise looped fibers from both precursor webs. Therefore, tufts **6** can be, in a sense, either "punched through" second precursor web **21** or "pushed into" the tufts of second precursor web 21. In either case, it can be said that first and second precursor webs can be "locked" in place by frictional engagement with openings **4.** In some embodiments, for example, the lateral width of opening **4** (i.e., the dimension measured parallel to its transverse axis) can be less than the maximum width of the tooth that formed the opening (per the process described below). This indicates a certain amount of recovery at the opening that tends to constrain tuft **6** from pulling back out through opening **4.** The frictional engagement of the tufts and openings provides for a laminate web structure having permanent tufting on one side that can be formed without adhesives or thermal bonding.

Because in some embodiments at least one of the layers (e.g., a relatively low elongation polymer film or tissue paper second precursor web **21** in FIGS. 1-4) does not significantly contribute material to the tufts **6** (such as in the embodiments shown in FIGS. 1-4) a web **1** comprising a nonwoven first precursor web **20** can be characterized as being predominantly fibrous on both sides of web **1** with the fibers being contributed only by nonwoven first precursor web **20.** Therefore, tufts 6 can be spaced sufficiently closely so as to effectively cover first side **3** of web **1.** In such an embodiment, both sides of web **1** appear to be nonwoven, with a difference between the two sides **3** and **5** being a difference in surface texture. Therefore, in one embodiment, the invention can be described as a laminate material of two or more precursor webs, wherein both sides of the laminate web are substantially covered by fibers from only one of the precursor webs.

As shown in FIGS. 1-4, one characteristic of tufts **6** can be the predominant directional alignment of the fibers **8** or **18.** For example, looped, aligned fibers **8** can be described as having a significant or major vector component parallel to the Z-CD plane and the looped fibers **8** have a substantially uniform alignment with respect to transverse axis **T** when viewed in plan view, such as in FIG. 4. By "looped" fibers **8** is meant fibers **8** that are integral with and begin and end in first precursor web **20** but extend outwardly in the Z-direction from first surface **13** of second precursor web **21.** By "aligned" with respect to looped fibers **8** of tufts **6** is meant that looped fibers **8** are all generally oriented such that, if viewed in plan view as in FIG. 4, each of the looped fibers **8** has a significant vector component parallel to the transverse axis **T,** and preferably a major vector component parallel to the transverse axis **T.**

In contrast, non-looped fibers **18** are integral with, but only begin in first precursor web **20** and have a free end extending outwardly in the Z-direction from first surface **13** of second precursor web **21.** Loose fibers **18** can also have a generally uniform alignment described as having a significant or major vector component parallel to the Z-CD plane.

For both looped fibers **8** and loose fibers **18,** the alignment is a characteristic of tufts **6** prior to any post-manufacture deformation due to winding onto a roll, or compression in use in an article of manufacture.

As used herein, a looped fiber **8** oriented at an angle of greater than 45 degrees from the longitudinal axis **L** when viewed in plan view, as in FIG. 4, has a significant vector component parallel to the transverse axis **T.** As used herein, a looped fiber **8** oriented at an angle of greater than 60 degrees from longitudinal axis **L** when viewed in plan view, as in FIG. 4, has a major vector component parallel to the transverse axis **T.** In a preferred embodiment, at least 50%, more preferably at least 70%, and more preferably at least 90% of fibers **8** of tuft **6** have a significant, and more preferably, a major vector component parallel to transverse axis **T.** Fiber orientation can be determined by use of magnifying means if necessary, such as a microscope fitted with a suitable measurement scale. In general, for a non-linear segment of fiber viewed in plan view, a straight-line approximation for both longitudinal axis **L** and the looped fibers **8** can be used for determining the angle of looped fibers **8** from longitudinal axis **L.** For example, as shown in FIG. 4, one fiber **8a** is shown emphasized by a heavy line, and its linear approximation **8b** is shown as a dashed line. This fiber makes an angle of approximately 80 degrees with the longitudinal axis (measured counterclockwise from **L).**

The orientation of looped fibers **8** in the tufts **6** is to be contrasted with the fiber composition and orientation for first precursor web **20,** which, for nonwoven webs is best described as having a substantially randomly-oriented fiber alignment. In a woven web embodiment, the orientation of the looped fibers **8** in tufts **6** could be the same as described above, but the fibers of first precursor web **20** would have the orientation associated with the particular weaving process used to make the web, e.g., a square weave pattern.

In the embodiment shown in FIG. 1 the longitudinal axes **L** of tufts **6** are generally aligned in the **MD.** Tufts **6** and, therefore, longitudinal axes **L,** can, in principle, be aligned in any orientation with respect to the **MD** or **CD.** Therefore, in general, it can be said that for each tuft **6,** the looped aligned fibers **8** are aligned generally orthogonal to the longitudinal axis **L** such that they have a significant vector component parallel to transverse axis **T,** and more preferably a major vector component parallel to transverse axis **T.**

In some embodiments, due to the preferred method of forming tufts **6,** as described below, another characteristic of tufts **6** comprising predominantly looped, aligned fibers **8,** can be their generally open structure characterized by open void area **10** defined interiorly of tufts **6,** as shown in FIGS. 2 and 3. The void area 10 may have a shape that is wider or larger at the distal end 31 of the tuft 6 and narrower at the base 17 of the tuft **6.** This is opposite to the shape of the tooth which is used to form the tuft 6. By "void area" is not meant an area completely free of any fibers; the term is meant as a general description of the general appearance of tufts **6.** Therefore, it may be that in some tufts **6** a loose fiber **18** or a plurality of loose fibers **18** may be present in the void area **10.** By "open" void area is meant that the two longitudinal ends of tuft **6** are generally open and free of fibers, such that tuft **6** can form something like a "tunnel" structure in an uncompressed state, as shown in FIG. 3.

Additionally, as a consequence of a preferred method of making web **1,** the second side **5** of web **1** exhibits discontinuities **16** characterized by a generally linear indentation defined by formerly random fibers of the second surface **14** of first precursor web **20** having been urged directionally (i.e., in the "Z -direction" generally orthogonal to the **MD-CD** plane as shown in FIGS. 1 and 3) into tufts **6** by the teeth of the forming structure, described in detail below. The abrupt change of orientation exhibited by the previously randomly-oriented fibers of first precursor web **20** defines the discontinuity **16,** which exhibits a linearity such that it can be described as having a longitudinal axis generally parallel to longitudinal axis **L** of the tuft **6.** Due to the nature of many nonwoven webs useful as first precursor webs **20,** discontinuity **16** may not be as distinctly noticeable as tufts **6.** For this reason, the discontinuities **16** on the second side **5** of web **1** can go unnoticed and may be generally undetected unless web **1** is closely inspected. As such, the second side **5** of web **1** can have the look and feel of an un-tufted first precursor web **20.** Thus in some embodiments, web **1** can have the textured look and feel of terry cloth on first side **3,** and a relatively smooth, soft look and feel on second side **5,** both sides being comprised of fibers from the same nonwoven web, i.e., the first precursor web **20.** In other embodiments, discontinuities **16** can appear as apertures, and may be apertures through web **1** via the ends of the tunnel-like tufts **6.**

From the description of web **1** comprising a nonwoven first precursor web **20,** it can be seen that the fibers **8** or **18** of tuft **6** can originate and extend from either the first surface **12** or the second surface **14** of first precursor web **20.** Of course the fibers **8** or **18** of tuft **6** can also extend from the interior **28** of first precursor web **20.** As shown in FIG. 3, the fibers **8** or **18** of tufts **6** extend due to having been urged out of the generally two-dimensional plane of first precursor web **20** (i.e., urged in the "Z -direction" as shown in FIG. 3). In general, the fibers **8** or **18** of the tufts **6** comprise fibers that are integral with and extend from the fibers of the first precursor web **20.**

Therefore, from the above description, it is understood that in one embodiment web **1** can be described as being a laminate web formed by selective mechanical deformation of at least a first and second precursor webs, at least the first precursor web being a nonwoven web, the laminate web having a first side, the first side comprising the second precursor web and a plurality of discrete tufts, each of the discrete tufts comprising a plurality of tufted fibers being integral extensions of the first precursor web and extending through the second precursor web; and a second side, the second side comprising the first precursor web.

The extension of fibers **8** or **18** can be accompanied by a general reduction in fiber cross sectional dimension (e.g., diameter for round fibers) due to plastic deformation of the fibers and Poisson's ratio effects. Therefore, the aligned looped fibers **8** of tuft **6** can have an average fiber diameter less than the average fiber diameter of the fibers of first precursor web **20.** It is believed that this reduction in fiber diameter contributes to the perceived softness of the first side **3** of web **1,** a softness that can be comparable to cotton terry cloth, depending on the material properties of the first precursor web **20.** It has been found that the reduction in fiber cross-sectional dimension is greatest intermediate the base **17** and the distal portion **31** of tuft **6.** This is believed to be due to the preferred method of making, as disclosed more fully below. Briefly, as shown on FIG. 3, it is believed that portions of fibers at the base **17** and distal portion **31** of tufts **6** are adjacent the tip of teeth **110** of roll **104,** described more fully below, and are frictionally locked and immobile during processing. Thus, the intermediate portions of tufts **6** are more free to stretch, or elongate, and accordingly, can experience a corresponding fiber cross sectional dimension reduction. Some fibers of first precursor web **20** may laterally squeeze the base **17** of the tuft **6.** The base **17** of the tuft 6 may even be closed (if the fibers from the tuft 6 are close enough together to touch) or may remain open. Generally, any opening at the base **17** is narrow. The closing or narrowing or squeezing of other fibers at the base **17** can help to stabilize the tufts **6** and second precursor web **21.**

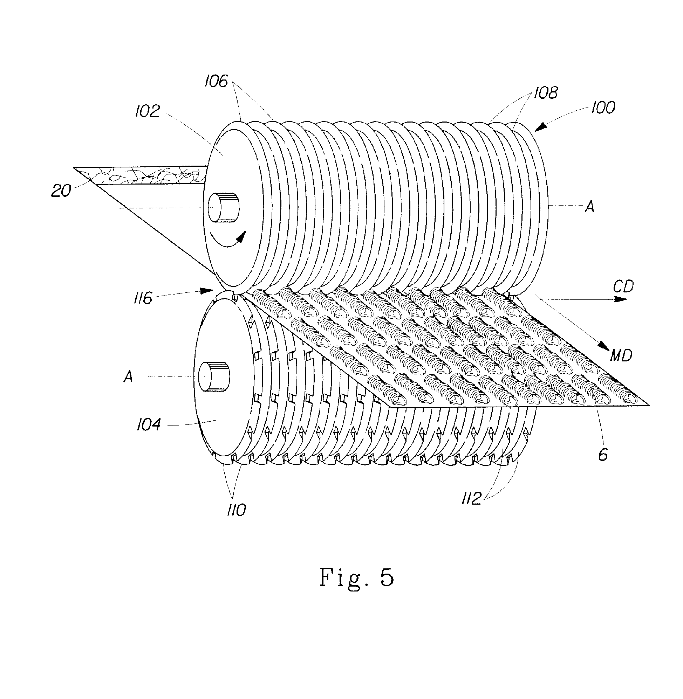

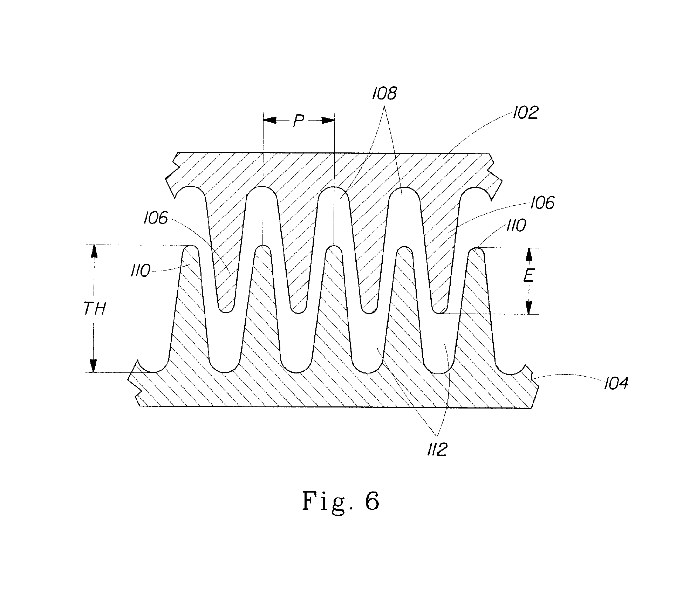

Referring to FIG. 5 there is shown an apparatus and method for making web **1** of the present invention. The apparatus **100** comprises a pair of intermeshing rolls **102** and **104,** each rotating about an axis **A,** the axes **A** being parallel in the same plane. Roll **102** comprises a plurality of ridges **106** and corresponding grooves **108** which extend unbroken about the entire circumference of roll **102.** Roll **104** is similar to roll **102,** but rather than having ridges that extend unbroken about the entire circumference, roll **104** comprises a plurality of rows of circumferentially-extending ridges that have been modified to be rows of circumferentially-spaced teeth **110** that extend in spaced relationship about at least a portion of roll **104.** The individual rows of teeth **110** of roll **104** are separated by corresponding grooves **112.** In operation, rolls **102** and **104** intermesh such that the ridges **106** of roll **102** extend into the grooves **112** of roll **104** and the teeth **110** of roll **104** extend into the grooves **108** of roll **102.** The intermeshing is shown in greater detail in the cross sectional representation of FIG. 6, discussed below. Both or either of rolls **102** and **104** can be heated by means known in the art such as by using hot oil filled rollers or electrically-heated rollers.

In FIG. 5, the apparatus **100** is shown in a preferred configuration having one patterned roll, e.g., roll **104,** and one non-patterned grooved roll **102.** However, in certain embodiments it may be preferable to use two patterned rolls **104** having either the same or differing patterns, in the same or different corresponding regions of the respective rolls. Such an apparatus can produce webs with tufts **6** protruding from both sides of the web **1.** An apparatus could also be designed to have teeth that pointing in opposite directions on the same roll. This would result in a web with tufts 6 being produced on both sides of the web.

The method of making a web **1** of the present invention in a commercially viable continuous process is depicted in FIG. 5. Web **1** is made by mechanically deforming precursor webs, such as first and second precursor webs, **20** and **21** that can each be described as generally planar and two dimensional prior to processing by the apparatus shown in FIG. 5. By "planar" and "two dimensional" is meant simply that the webs start the process in a generally flat condition relative to the finished web **1** that has distinct, out-of-plane, Z-direction three-dimensionality due to the formation of tufts **6.** "Planar" and "two-dimensional" are not meant to imply any particular flatness, smoothness or dimensionality.

The process and apparatus of the present invention is similar in many respects to a process described in U.S. Pat. No. 5,518,801 entitled "Web Materials Exhibiting Elastic-Like Behavior" and referred to in subsequent patent literature as "SELF" webs, which stands for "Structural Elastic-like Film". However, there are significant differences between the apparatus and process of the present invention and the apparatus and process disclosed in the '801 patent, and the differences are apparent in the respective webs produced thereby. As described below, the teeth **110** of roll **104** have a specific geometry associated with the leading and trailing edges that permit the teeth to essentially "punch" through the precursor webs **20, 21** as opposed to, in essence, deforming the web. In a two layer laminate web **1** the teeth **110** urge fibers from a first precursor web **20** simultaneously out-of-plane and through second precursor web **21,** which is punctured, so to speak, by the teeth **110** pushing the fibers **8** through to form tufts **6.** Therefore, a web **1** of the present invention can have tufts **6** of loose fiber ends **18** and/or "tunnel-like" tufts **6** of looped, aligned fibers **8** extending through and away from the surface **13** of a first side 3, unlike the "tent-like" rib-like elements of SELF webs which each have continuous side walls associated therewith, i.e., a continuous "transition zone," and which do not exhibit interpenetration of one layer through another layer.

Precursor webs **20** and **21** are provided either directly from their respective web making processes or indirectly from supply rolls (neither shown) and moved in the machine direction to the nip **116** of counter-rotating intermeshing rolls **102** and **104.** The precursor webs are preferably held in a sufficient web tension so as to enter the nip 16 in a generally flattened condition by means well known in the art of web handling. As each precursor web **20, 21** goes through the nip **116** the teeth **110** of roll **104** which are intermeshed with grooves **108** of roll **102** simultaneously urge portions of first precursor web **20** out of the plane of first precursor web **20** and through second precursor web **21** to form tufts **6.** In effect, teeth **110** "push" or "punch" fibers of first precursor web **20** through second precursor web **21.**

As the tip of teeth **110** push through first and second precursor webs **20, 21** the portions of the fibers of first precursor web **20** that are oriented predominantly in the **CD** across teeth **110** are urged by the teeth **110** out of the plane of first precursor web **20.** Fibers can be urged out of plane due to fiber mobility, or they can be urged out of plane by being stretched and/or plastically deformed in the Z-direction. Portions of first precursor web **20** urged out of plane by teeth **110** push through second precursor web **21,** which due to its relatively lower extensibility, ruptures, thereby resulting in formation of tufts **6** on first side **3** of web **1.** Fibers of first precursor web **20** that are predominantly oriented generally parallel to the longitudinal axis **L,** i.e., in the **MD** of precursor web **20** as shown in FIG. 1, are simply spread apart by teeth **110** and remain substantially in their original, randomly-oriented condition. This is why the looped fibers **8** can exhibit the unique fiber orientation in embodiments such as the one shown in FIGS. 1-4, which is a high percentage of fibers of each tuft **6** having a significant or major vector component parallel to the transverse axis **T** of tuft **6.**

It can be appreciated by the forgoing description that when web **1** is made by the apparatus and method of the present invention that the precursor webs **20, 21** can possess differing material properties with respect to the ability of the precursor webs to elongate before failure, e.g., failure due to tensile stresses. In particular, a nonwoven first precursor web **20** can have greater fiber mobility and/or greater fiber elongation characteristics relative to second precursor web **21,** such that the fibers thereof can move or stretch sufficiently to form tufts **6** while the second precursor web **21** ruptures, i.e., does not stretch to the extent necessary to form tufts. However, in other embodiments, both precursor webs have sufficient elongation such that fibers thereof can move or stretch sufficiently to form tufts **6.**

The degree to which the fibers of nonwoven precursor webs are able to extend out of plane without plastic deformation can depend upon the degree of inter-fiber bonding of the precursor web. For example, if the fibers of a nonwoven precursor web are only very loosely entangled to each other, they will be more able to slip by each other (i.e., to move relative to adjacent fibers by reptation) and therefore be more easily extended out of plane to form tufts. On the other hand, fibers of a nonwoven precursor web that are more strongly bonded, for example by high levels of thermal point bonding, hydroentanglement, or the like, will more likely require greater degrees of plastic deformation in extended out-of-plane tufts. Therefore, in one embodiment, first precursor web **20** can be a nonwoven web having relatively low inter-fiber bonding, and second precursor web **21** can be a nonwoven web having relatively high inter-fiber bonding, such that the fibers of first precursor web can extend out of plane, while the fibers of second precursor web **21** cannot. Upon sufficient force applied to first precursor web **21,** the fibers therein tend to extend, while the fibers of second precursor web, unable to extend, tend to break.

The number, spacing, and size of tufts **6** can be varied by changing the number, spacing, and size of teeth **110** and making corresponding dimensional changes as necessary to roll **104** and/or roll **102.** This variation, together with the variation possible in precursor webs **20, 21** permits many varied webs **1** to be made for many purposes. For example, web **1** made from a first precursor web **20** comprising a relatively high basis weight woven fabric having **MD** and **CD** woven plastically-extensible threads and a second precursor web **21** comprising relatively high basis weight, relatively low-extensible synthetic polymer nonwoven material could be made into a strong, porous ground covering, such as an erosion control device useful for reducing sloping path deterioration and enabling growth of indigenous vegetation in unstable soil.

FIG. 6 shows in cross section a portion of the intermeshing rolls **102** and **104** and ridges **106** and teeth **110.** As shown teeth **110** have a tooth height **TH** (note that **TH** can also be applied to ridge height; in a preferred embodiment tooth height and ridge height are equal), and a tooth-to-tooth spacing (or ridge-to-ridge spacing) referred to as the pitch **P.** As shown, depth of engagement **E** is a measure of the level of intermeshing of rolls **102** and **104** and is measured from tip of ridge **106** to tip of tooth **110.** The depth of engagement **E,** tooth height **TH,** and pitch **P** can be varied as desired depending on the properties of precursor webs **20, 21** and the desired characteristics of web 1. For example, in general, the greater the level of engagement **E,** the greater the necessary elongation or fiber-to-fiber mobility characteristics the fibers of first precursor web **20** must possess. Also, the greater the density of tufts **6** desired (tufts **6** per unit area of web **1),** the smaller the pitch should be, and the smaller the tooth length **TL** and tooth distance **TD** should be, as described below.

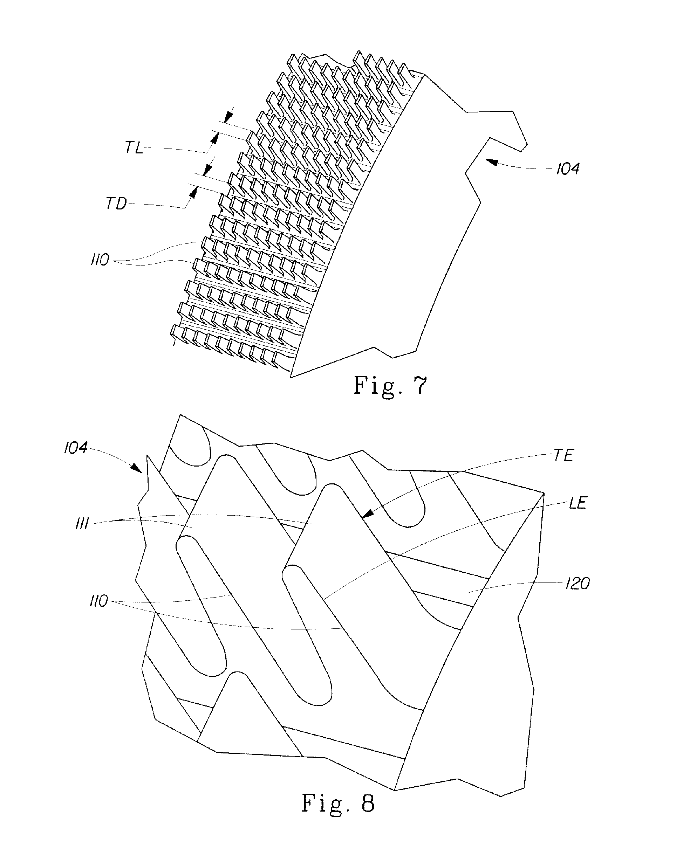

FIG. 7 shows one embodiment of a roll **104** having a plurality of teeth **110** useful for making a terry cloth-like web **1** from a nonwoven first precursor web **20** having a basis weight of between about 60 gsm and 100 gsm, preferably about 80 gsm and a polyolefinic film (e.g., polyethylene or polypropylene) second precursor web **21** having a density of about 0.91-0.94 and a basis weight of about 20 gsm.

An enlarged view of teeth **110** is shown in FIG. 8. In this embodiment of roll **104** teeth **110** have a uniform circumferential length dimension **TL** measured generally from the leading edge **LE** to the trailing edge **TE** at the tooth tip **111** of about 1.25 mm and are uniformly spaced from one another circumferentially by a distance **TD** of about 1.5 mm. For making a terry-cloth web **1** from web **1** having a total basis weight in the range of about 60 to about 100 gsm, teeth **110** of roll **104** can have a length **TL** ranging from about 0.5 mm to about 3 mm and a spacing **TD** from about 0.5 mm to about 3 mm, a tooth height **TH** ranging from about 0.5 mm to about 5 mm, and a pitch **P** between about 1 mm (0.040 inches) and about 5 mm (0.200 inches). Depth of engagement **E** can be from about 0.5 mm to about 5 mm (up to a maximum equal to tooth height **TH).** Of course, **E, P, TH, TD** and **TL** can be varied independently of each other to achieve a desired size, spacing, and area density of tufts **6** (number of tufts **6** per unit area of web **1).**

As shown in FIG. 8, each tooth **110** has a tip **111,** a leading edge **LE** and a trailing edge **TE.** The tooth tip **111** is elongated and has a generally longitudinal orientation, corresponding to the longitudinal axes **L** of tufts **6** and discontinuities **16.** It is believed that to get the tufted, looped tufts **6** of the web **1** that can be described as being terry cloth-like, the **LE** and **TE** should be very nearly orthogonal to the local peripheral surface **120** of roll **104.** As well, the transition from the tip **111** and **LE** or **TE** should be a sharp angle, such as a right angle, having a sufficiently small radius of curvature such that teeth **110** push through second precursor web **21** at the **LE** and **TE.** Without being bound by theory, it is believed that having relatively sharply angled tip transitions between the tip of tooth **110** and the **LE** and **TE** permits the teeth **110** to punch through precursor webs **20, 21** "cleanly", that is, locally and distinctly, so that the first side **3** of the resulting web **1** can be described as "tufted" rather than "deformed." When so processed, the web **1** is not imparted with any particular elasticity, beyond what the precursor webs **20** and **21** may have possessed originally. The punching through of the precursor web 21 may result in a small portion of the web 21 forming "confetti" or small pieces.

While not wishing to be bound by theory, it is believed that if the fibers of the precursor webs have a highly curvilinear shape, e.g., curled fibers, the resultant tufts **6** will have more looped fibers **8** and less broken fibers **18** as compared to more linear fiber conformations. It is believed that such fiber conformations have a lesser chance of bridging between two adjacent teeth, and, as a result they are less prone to be stretched beyond their breaking point, and thus have a greater chance of forming complete loop structures. Furthermore, such curvilinear-shaped fibers can be made by using eccentric bicomponent fibers, or side-by-side bicomponent fibers, such as bicomponent fibers consisting of polyethylene and nylon.

In preferred embodiments first and second precursor webs are nonwoven webs in which there are minimal fiber-to-fiber bonds. For example, the precursor web can be a nonwoven web having a pattern of discrete thermal point bonds, as is commonly known in the art for nonwoven webs. In general, however, it is desirable to minimize the number of bond points and maximize the spacing so as to allow for maximum fiber mobility and dislocation at during formation of tufts **6.** In general, utilizing fibers having relatively high diameters, and/or relatively high extension to break, and/or relatively high fiber mobility, results in better and more distinctly formed tufts **6.**

Although web 1 is disclosed in preferred embodiments as a two layer web made from two precursor webs, it is not necessary that it be limited to two layers. For example, a three-layer or more laminate can be made from three precursor webs, as long as one of the precursor webs can extend and push through openings in another layer to form tufts. For example, web 1 could comprise the top sheet, secondary topsheet, and core of hygiene products. In general, it is not necessary that adhesive or other bonding means be utilized to make laminate web **1.**

The constituent layers of web **1** (e.g., precursor webs **20** and **21** and any other layers) can be held in a face-to-face laminated relationship by virtue of the "locking" effect of the tufts **6** that extend through openings **4** in second precursor web **21.** In some embodiments it may be desirable to use adhesives or thermal bonding or other bonding means, depending on the end use application of web **1.** For example, a web **1** comprising bicomponent fiber nonwoven webs can be through-air bonded after formation of tufts **6** to provide for layer-to-layer adhesion for greater peel strength. Additionally, it may be desirable to apply adhesive to at least a portion of one of the precursor webs. For example, in some embodiments adhesive, chemical bonding, resin or powder bonding, or thermal bonding between layers can be selectively applied to certain regions or all of the precursor webs. In the case of adhesive application, for example, adhesive can be applied in a continuous manner, such as by slot coating, or in a discontinuous manner, such as by spraying, extruding, and the like. Discontinuous application of adhesive can be in the form of stripes, bands, droplets, and the like.

In a multilayer web **1** each precursor web can have different material properties, thereby providing web **1** with beneficial properties when used as a topsheet in a disposable absorbent article such as a sanitary napkin. For example, web **1** comprising two (or more) precursor webs, e.g., first and second precursor webs, can have beneficial fluid handling properties. For superior fluid handling, for example, first precursor web **20** can be comprised of relatively hydrophilic fibers. Second precursor web **21** can be comprised of relatively hydrophobic fibers. The tufts **6** of such a web could form a topsheet having a relatively hydrophobic body-facing surface, with hydrophilic tufts to pull fluid away from the body and through the topsheet. Fluid deposited upon the upper, relatively hydrophilic tufts can be quickly transported away from the relatively hydrophobic layer to the portion of the article underlying the second precursor web layer (e.g., the absorbent core). Without being bound by theory, it is believed that one reason for the observed rapid fluid transport is the capillary structures formed by the generally aligned fibers **8, 18** of tufts **6.** The fibers **8, 18** form directionally-aligned capillaries between adjacent fibers, and the capillary action is enhanced by the general convergence of fibers near the base **17** of tufts **6.**

It is believed that the rapid fluid transport is further increased due to the ability of fluid to enter the web **1** via the voids **10** defined by looped tufts **6.** This "lateral entry" capability and/or capillary action, and/or the hydrophilicity gradient afforded by the structure of web **1** makes web **1** an ideal material for optimal fluid handling for disposable absorbent articles. In particular, a multilayer web **1** can provide for even greater improvement in fluid handling characteristics.

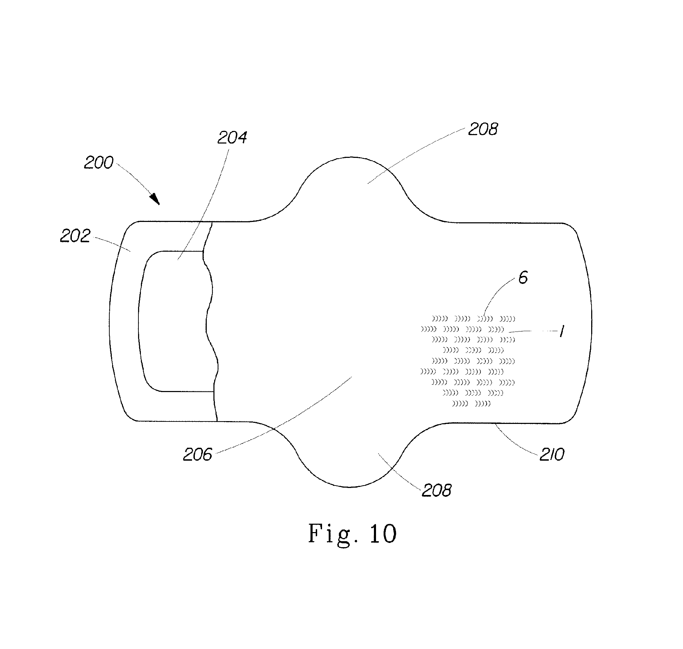

FIG. 10 shows in partial cut away plan view a sanitary napkin having as one of its components a web **1** of the present invention. In general, sanitary napkin **200** comprises a backsheet **202,** a topsheet **206** and an absorbent core **204** disposed between the topsheet **206** and backsheet **202** which can be joined about a the periphery **210.** Sanitary napkin **1** can have side extensions, commonly referred to as "wings" **208** designed to wrap the sides of the crotch region of the panties of the user of sanitary napkin **1.** Sanitary napkins, including topsheets for use as the body facing surface thereof, are well known in the art and need no detailed description of various alternative and optional designs. In addition to sanitary napkins, web **1** can also be used in a diaper or adult incontinence product or other disposable hygiene products. However, it is noted that web **1** can be used as, or as a component of, one or more of a backsheet, core material, topsheet, secondary topsheet, or wing material. Web **1** can also have multiple layers and comprise a topsheet, secondary topsheet, core, backsheet, or any number of layers.

Web **1** is especially useful as a topsheet **206** of sanitary napkin **200.** Web **1** is particularly beneficial as a topsheet **206** for sanitary napkins due to the combination of excellent fluid acquisition and distribution to the absorbent core **204,** and excellent prevention of rewet to the body-facing surface of topsheet **206** when in use. Rewet can be a result of at least two causes: (1) squeezing out of the absorbed fluid due to pressure on the sanitary napkin **200;** and/or (2) wetness entrapped within or on the topsheet **206.** In a preferred topsheet **206** both properties, fluid acquisition and fluid retention, are maximized and rewet is minimized. Said differently, preferably a topsheet will exhibit high rates of fluid acquisition, and low levels of rewet.

A topsheet **206** can be made by using a nonwoven first precursor web **20** and a fluid impermeable polyethylene film second precursor web **21.** The basis weights of the component webs can be varied, however, in general due to cost and benefit considerations a total basis weight of between about 20 gsm and 80 gsm is desirable for web **1.** When a sanitary napkin is used having a topsheet **206** comprising web **1** with first side **3** being the body-facing side, and the second side **5** being in fluid communication with an underlying absorbent core, fluid can be acquired by tufts **6** on first side **3** of web **1** and wicked through second precursor web **21** to second side **5** of web **1** which can then be desorbed to the absorbent core **204.** Because tufts **6** are discrete and spaced apart, and are separated by a fluid impermeable second precursor web **21,** rewet can be minimized. Alternatively, web **1** could be used with first side **3** being the fluid communication side and second side **5** being the body-facing side. This enables the discontinuities **16** to potentially allow fluid to be transported into or through the tufts **6.**

In a sanitary napkin of the present invention, topsheet **206** has applied thereto a semi-solid lotion composition. The lotion composition can be any of known lotions, such as lotions comprising petrolatum, which can provide a skin benefit to the user. In a preferred embodiment, the lotion also provides a clean body benefit to the user. That is, the lotion preferably coats the body and renders menses less susceptible to sticking to the body, including hair and skin. Preferably, therefore, the lotion is hydrophobic, and renders hair and skin hydrophobic.

Lotion can be applied in any manner known in the art for applying lotions to nonwoven webs. Lotion can be applied to the tips (i.e., the distal ends) of tufts **6.** It has been found that applying lotion to the tips enables efficient transfer to the skin of the wearer. Without being bound by theory, it is believed that the tufts act as little brushes to wipe the lotion onto the body during motion, such as walking.

The lotion of the present invention can include those disclosed in US 5,968,025; US 6,627,787; US 6,498,284; US 6,426,444; US 6,586,652; US 3,489,148; US 6,503,526; 6,287,581; US 6,475,197; US 6,506,394; US 6,503,524; US 6,626,961; US 6,149,934; US 6,515,029; US 6,534,074; US 6,149,932WO 2000038747; or EP-A 927,050.

In addition to (or instead of) lotion treatments, the topsheet **206** (or portions thereof) can be treated with other materials or compositions to render it sufficiently hydrophobic. For example, the topsheet can be treated with silicone treatments, low surface energy treatments, fluorinated hydrocarbon treatments. In general, relatively hydrophobic means a material or composition having a contact angle with water of at least about 70 degrees, preferably at least about 90 degrees. In general, low surface energy means between about 20 and about 60 dynes per square centimeter, preferably from about 20 to about 50 dynes per square centimeter, and more preferably from about 20-40 dynes per square centimeter.

In a preferred embodiment, web **1** is used as a topsheet **206** in conjunction with a high capacity and highly absorbent core **204.** In general, a preferred absorbent core is an airlaid core of the type disclosed in US 5,445,777; or US 5,607,414. In a preferred embodiment, absorbent core **204** is the type generally referred to as HIPE foams, such as those disclosed in US 5,550,167; US, 5,387,207; US 5,352,711; and 5,331,015. In a preferred embodiment, absorbent core **204** has a capacity after desorption at 30 cm of less than about 10% of its free absorbent capacity; a capillary absorption pressure of from about 3 to about 20 cm; a capillary desorption pressure of from about 8 to about 25 cm; a resistance to compression deflection of from about 5 to about 85% when measured under a confining pressure of 0.74 psi; and a free absorbent capacity of from about 4 to 125 grams/gram. Each of these parameters can be determined as set forth in US 5,550,167. issued August 27, 1996 to DesMarais. One advantage of utilizing the airlaid or HIPE foam cores as disclosed is that the absorbent core can be made very thin. For example, an absorbent core of the present invention can have an average caliper (thickness) of less than about 20 mm, preferably less than about 10 mm, and the thickness can be less than about 5 mm.

As can be understood from the above description of webs **1** and apparatus **100** of the present invention, many various structures of webs **1** can be made without departing from the scope of the present invention as claimed in the appended claims. For example, topsheet **206** can additionally be coated or treated with medicaments, cleaning fluids, anti-bacterial solutions, emulsions, fragrances, or surfactants. Likewise, apparatus **100** can be configured to only form tufts **6** on a portion of the web **1,** or to form varying sizes or area densities of tufts **6.**

Another advantage of the process described to produce the webs of the present invention is that the webs can be produced in-line with other web production equipment or in-line with disposable absorbent article production equipment. Additionally, there may be other solid state formation processes that can be used either prior to or after the process of the present invention. For example, a web could be processed according to the present invention and then apertured with a stretching process, such as one described in US Patent No. 5,658,639 to Curro et al. Alternatively, a material could be made into a composite through a variety of processes, such as one described in US Publication No. 2003/028,165A1 to Curro et al. or ring rolled, for example as in US Patent No. 5,167,897 to Weber et al. and then processed according to the present invention. The resulting webs can thus exhibit the combined benefits of these multiple material modifications.

All documents cited in the Detailed Description of the Invention are, in relevant part, incorporated herein by reference; the citation of any document is not to be construed as an admission that it is prior art with respect to the present invention. To the extent that any meaning or definition of a term in this written document conflicts with any meaning or definition of the term in a document incorporated by reference, the meaning or definition assigned to the term in this written document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A sanitary napkin **characterized by**:
a topsheet having a body-facing side and comprising a plurality of discrete tufts of fibrous material,
a lotion composition applied to at least a portion of said body-facing side of said topsheet, and
an absorbent core in fluid communication with said topsheet, said absorbent core having an average thickness of less than about 10 mm, and a free absorbent capacity of from about 4 to about 125 grams per gram.

2. The sanitary napkin of Claim 1, wherein said topsheet comprises a nonwoven web.

3. The sanitary napkin of Claims 1 or 2, wherein said topsheet comprises a polymer film web.

4. The sanitary napkin of any of the preceding claims, wherein said topsheet comprises a nonwoven web and a polymer film web.

5. The sanitary napkin of any of the preceding claims, wherein said topsheet comprises at least two precursor webs, and said discrete tufts comprise fibers each said precursor web.

6. The sanitary napkin of any of the preceding claims, wherein said topsheet comprises a first precursor web and a second precursor web, wherein said first precursor web comprises a nonwoven web and said second precursor web comprises a polymer film, and wherein said tufts comprise fibers from said nonwoven web first precursor web.

7. A sanitary napkin **characterized by**:
a topsheet having a body-facing side and comprising from 10 to 50 discrete tufts of fibrous material per square centimeter;
a semi-solid lotion composition applied to at least a portion of said body-facing side of said topsheet; and
an absorbent core in fluid communication with said topsheet, said absorbent core being a HIPE foam and having an average thickness of less than about 10 mm.

8. The sanitary napkin of Claim 7, wherein said lotion comprises petrolatum.

9. The sanitary napkin of Claims 7 or 8, wherein said topsheet comprises a first precursor web and a second precursor web, wherein at least one of said precursor webs is relatively more hydrophobic with respect to said other of said precursor webs.

10. A sanitary napkin **characterized by**:
a topsheet comprising a first precursor web and a second precursor web, wherein at least one of said precursor webs is relatively more hydrophobic with respect to said other of said precursor webs, and wherein said topsheet further comprises discrete tufts of fibrous material from said first or second precursor webs;
a semi-solid hydrophobic lotion composition comprising petrolatum disposed on at least a portion of said topsheet; and
an absorbent core in fluid communication with said topsheet, said absorbent core having an average thickness of less than about 7 mm; and
a backsheet joined to said topsheet.
